# FASCICULE DE BREVET EUROPEEN

(11) **EP 3 010 450 B1**
(45) Date de publication et mention de la délivrance du brevet: **23.08.2017**
(21) Numéro de dépôt: 14739886.1
(22) Date de dépôt: 20.06.2014
(51) Int. Cl.: A61F 2/88, A61M 27/00

(54) **ECARTEUR DE TYPE STENT**
STENTABSTANDSHALTER
STENT SPACER

(30) Priorité: 21.06.2013 FR 1355935
(43) Date de publication de la demande: 27.04.2016
(73) Titulaire: Université Grenoble Alpes, 38400 Saint-Martin-d'Hères (FR); Centre National de la Recherche Scientifique, 75794 Paris Cedex 16 (FR); Assistance Publique Hôpitaux de Paris, 75004 Paris 4 (FR)
(72) Inventeur: FAVIER, Denis, 38041 Grenoble Cedex 9 (FR); MOZER, Pierre, 75004 Paris 4 (FR); PAYAN, Yohan, 38580 Allevard (FR); ANTHERIEU, Gabriel, 38041 Grenoble Cedex 9 (FR); CONNESSON, Nathanael, 38041 Grenoble Cedex 9 (FR)
(74) Mandataire: Thibon, Laurent
(86) Numéro de dépôt international: PCT/FR2014/051547
(87) Numéro de publication internationale: WO 2014/202917

(56) Documents cités:
- WO-A1-2006/086304
- WO-A2-02/36045
- US-A1- 2002 142 119
- US-A1- 2013 041 454

## Description

### Domaine

La présente demande concerne le domaine des écarteurs ou extenseurs de façon générale, et vise plus particulièrement le domaine des écarteurs vasculaires ou urétraux habituellement désignés sous le nom de stent.

### Exposé de l'art antérieur

Pour maintenir ouverts et/ou agrandir la lumière de certains conduits ou vaisseaux du corps humain ou animal rétrécis ou obstrués par des maladies, il est connu d'insérer dans le conduit une prothèse de forme tubulaire habituellement désignée sous le nom de stent. Pendant une phase de positionnement du stent dans le conduit, le stent est dans une configuration contractée dans laquelle son diamètre est nettement inférieur au diamètre du conduit, ce qui permet son libre déplacement à l'intérieur du conduit. Une fois le stent positionné à l'endroit souhaité, il est prévu une étape d'expansion du stent, au cours de laquelle le stent est radialement étiré jusqu'à un diamètre du même ordre que celui du conduit voire légèrement supérieur à celui du conduit, de façon à agrandir l'ouverture de la zone rétrécie du conduit. Le stent peut être laissé en place le temps de traiter les causes du rétrécissement du conduit. Dans de nombreux cas, il doit cependant être retiré après le traitement.

Des exemples de stents à mémoire de forme sont notamment décrits dans la demande de brevet US20130041454.

Un inconvénient des stents connus réside dans le fait que leur retrait est relativement complexe et peut entrainer des lésions du conduit.

### Résumé

L'objet de l'invention est décrit dans la revendication 1. Un objet d'un mode de réalisation est de prévoir un écarteur palliant tout ou partie des inconvénients des écarteurs connus.

Un objet d'un mode de réalisation est de prévoir un écarteur de type stent plus simple à extraire d'un conduit que les stents connus.

Selon un mode de réalisation, le premier matériau à mémoire de forme a une première température d'activation, et le deuxième matériau à mémoire de forme a une deuxième température d'activation supérieure à la première température.

Selon un mode de réalisation, les première et deuxième parties sont agencées de façon que l'écarteur adopte une première configuration lorsque le premier matériau est activé, et une deuxième configuration lorsque le deuxième matériau est activé.

Selon un mode de réalisation, le premier matériau à mémoire de forme est un matériau super-élastique, et le deuxième matériau à mémoire de forme mémorise une forme activable thermiquement.

Selon un mode de réalisation, les première et deuxième parties sont agencées de façon que l'écarteur adopte une première configuration lorsque le deuxième matériau n'est pas activé, et une deuxième configuration lorsque le deuxième matériau est activé.

Selon un mode de réalisation, au moins un tronçon de l'écarteur a une forme générale approximativement cylindrique ayant un premier diamètre dans la première configuration et un deuxième diamètre inférieur au premier diamètre dans la deuxième configuration. Les première et deuxième parties font partie d'un même élément filiforme de forme générale hélicoïdale.

Selon un mode de réalisation, l'élément filiforme a la forme d'un diabolo.

Selon un mode de réalisation, la première partie est un fil creux, et la deuxième partie est un fil de plus petit diamètre disposé dans le fil creux.

Selon un mode de réalisation, les première et deuxième parties sont des zones distinctes de la section transversale d'un même fil.

Selon un mode de réalisation, les premier et deuxième matériaux à mémoire de forme sont des alliages à base de nickel et de titane.

Selon un mode de réalisation, l'écarteur comprend des bornes de contact permettant d'appliquer un courant électrique dans les première et/ou deuxième parties.

Selon un mode de réalisation, l'écarteur comprend en outre un revêtement en silicone.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 représente schématiquement le fonctionnement d'un exemple de stent ;
la figure 2 représente schématiquement le fonctionnement d'un mode de réalisation d'un stent ;
la figure 3 est une vue en coupe partielle illustrant plus en détail un exemple d'un mode de réalisation préféré d'un stent compatible avec le fonctionnement de la figure 2 ;
la figure 4 est une vue en coupe partielle illustrant plus en détail un autre exemple de réalisation d'un stent compatible avec le fonctionnement de la figure 2 ; et
la figure 5 est une vue en coupe illustrant un exemple de forme générale que peut avoir un stent du type décrit en relation avec les figures 2 à 4.

Par souci de clarté, de mêmes éléments ont été désignés par de mêmes références aux différentes figures. De plus, seuls les éléments utiles à la compréhension de l'invention ont été représentés et seront décrits. En particulier, les outils susceptibles d'être utilisés pour poser et/ou retirer un stent n'ont pas été décrits en détail ni représentés, les modes de réalisation décrits étant compatibles avec des outils usuels de pose et de retrait de stents, ou la réalisation d'outils de pose et de retrait de stents compatibles avec les modes de réalisation décrits étant à la portée de l'homme du métier.

### Description détaillée

On a déjà proposé de réaliser un stent en un matériau à mémoire de forme, c'est-à-dire un matériau ayant la capacité de mémoriser une forme et, après une déformation, d'y retourner presque instantanément lorsque sa température dépasse une température de transition ou d'activation.

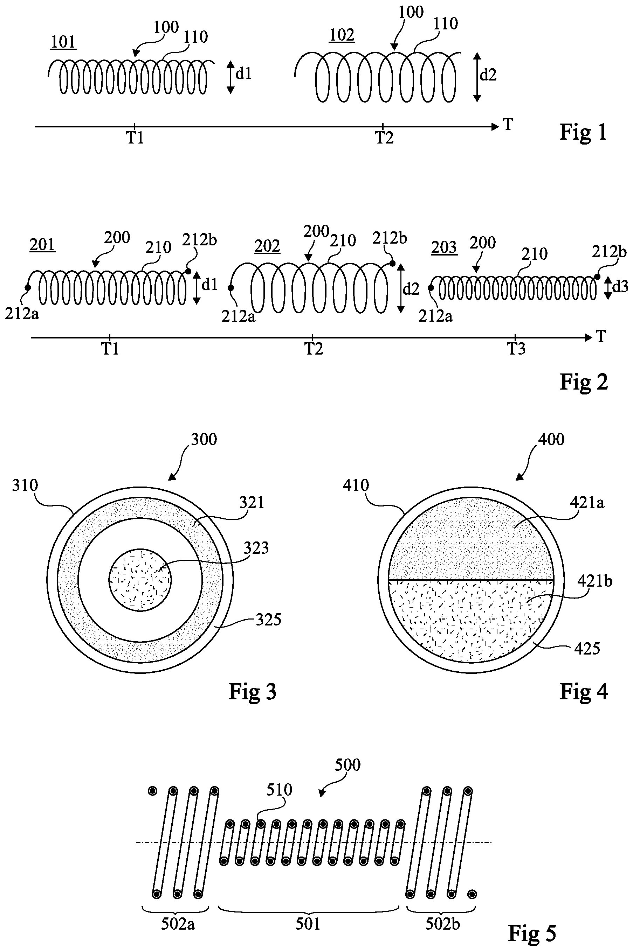

La figure 1 représente schématiquement le fonctionnement d'un exemple de stent à mémoire de forme. Le stent 100 de la figure 1 comprend un fil continu 110 de forme hélicoïdale en un matériau à mémoire de forme, par exemple un alliage nickel-titane, ayant une température d'activation T2.

Avant une phase d'insertion du stent dans un conduit (non représenté), le stent est conformé dans une configuration 101 dans laquelle le stent a une forme générale cylindrique de diamètre d1 inférieur au diamètre normal du conduit, c'est-à-dire au diamètre du conduit en l'absence de rétrécissement du à une maladie. Le stent est inséré dans le conduit dans cette configuration 101 et positionné à l'endroit souhaité, par exemple au moyen d'un cathéter (non représenté).

Une fois correctement positionné dans le conduit, le stent est expansé et adopte une configuration 102 dans laquelle le stent a une forme générale cylindrique de diamètre d2 supérieur au diamètre d1. Si la température T2 d'activation du matériau à mémoire de forme est inférieure à la température du corps humain ou animal dans lequel est introduit le stent, l'expansion peut être obtenue en expulsant le stent (dans sa forme activée) du cathéter, en exploitant la super-élasticité propre aux matériaux à mémoire de forme. Si la température T2 d'activation du matériau à mémoire de forme est supérieure à la température du corps humain ou animal dans lequel est introduit le stent, le stent peut, une fois correctement positionné dans le conduit, être chauffé à une température supérieure ou égale à la température T2. Pour cela, une solution aqueuse chauffée à une température supérieure ou égale à la température T2 peut être injectée dans le conduit.

Le diamètre d2 est par exemple du même ordre que le diamètre normal du conduit ou légèrement supérieur au diamètre normal du conduit, ce qui permet, le cas échéant, d'agrandir la lumière de la portion rétrécie du conduit au niveau du stent. La forme du stent dans la configuration 102 correspond à la forme mémorisée par le fil 110 à mémoire de forme, ou forme mémoire du stent. Le matériau à mémoire de forme peut être choisi tel que sa température d'activation T2 soit supérieure à la température du corps humain ou animal, par exemple de l'ordre de 40 à 45°C, pour éviter une activation non voulue du stent pendant son insertion dans le conduit. Après son activation, le stent reste dans la configuration 102 même lorsque sa température redescend en dessous de la température T2, en raison des propriétés d'hystérésis du matériau à mémoire de forme.

Pour retirer le stent après une phase de traitement de la maladie, on peut prévoir de dérouler en force le fil hélicoïdal le constituant. Toutefois, un inconvénient est que le risque de lésion du conduit lors de retrait est relativement élevé.

A titre de variante, pour retirer le stent, on peut prévoir de le refroidir jusqu'à une température T1 de désactivation, inférieure à la température T2, à laquelle le matériau à mémoire de forme reste dans la forme radialement étirée de la configuration 102, mais acquière une déformabilité plus grande qu'à la température du corps humain ou animal, permettant de retirer le stent par déroulement du fil hélicoïdal en réduisant les risques de lésion du conduit.

Le matériau à mémoire de forme peut être choisi tel que sa température T1 de désactivation soit nettement inférieure à la température du corps humain ou animal, par exemple de l'ordre de 25°C, pour éviter une désactivation non souhaitée du stent. Pour refroidir le stent, on peut injecter une solution aqueuse à basse température (inférieure ou égale à T1) dans le voisinage du stent, par exemple par l'intermédiaire d'un cathéter. Toutefois, un inconvénient est que l'injection d'un fluide froid dans le voisinage du stent est une opération relativement complexe et peut poser problème.

Un objet des modes de réalisation décrits est de prévoir un stent à mémoire de forme palliant tout ou partie des inconvénients susmentionnés.

La figure 2 représente schématiquement le fonctionnement d'un mode de réalisation d'un stent 200. Le stent 200 comprend un élément filiforme 210 conformé selon une disposition générale sensiblement hélicoïdale, présentant des propriétés de mémoire de forme.

Avant une phase d'insertion du stent dans un conduit (non représenté), le stent est conformé dans une configuration 201 dans laquelle le stent a une forme générale cylindrique de diamètre d1 inférieur au diamètre normal du conduit. Le stent est inséré dans le conduit avec cette configuration 201 et positionné à un endroit souhaité du conduit, par exemple au moyen d'un cathéter (non représenté).

Une fois correctement positionné dans le conduit, le stent est chauffé à une température supérieure ou égale à une première température d'activation T2 de l'élément 210. Pour cela, un fluide chauffé à une température supérieure ou égale à la température T2 peut être injecté dans le conduit. Toutefois, dans un mode de réalisation préféré, on prévoit de chauffer le stent par effet joule en faisant circuler un courant électrique dans l'élément 210. Ceci permet un chauffage plus simple à mettre en oeuvre et plus efficace qu'un chauffage par injection de fluide. De plus, ceci permet de limiter les risques de brulures par rapport à un chauffage par injection de fluide. Pour permettre l'application d'un courant électrique de chauffage, l'élément 210 peut comporter deux bornes de contact 212a et 212b respectivement reliées à ses deux extrémités. Le courant électrique de chauffage est par exemple fourni par une pile ou batterie couplée à un outil d'insertion du stent dans le conduit. En fonction de l'utilisation envisagée, un fil conducteur (non visible sur les figures) peut être prévu pour ramener l'une des bornes de contact 212a, 212b à proximité de l'autre, pour faciliter l'application du courant électrique de chauffage.

Lorsque le stent atteint la température T2, il adopte une configuration 202 dans laquelle le stent a une forme générale cylindrique de diamètre d2 supérieur au diamètre d1. Le diamètre d2 est par exemple du même ordre de grandeur que le diamètre normal du conduit ou légèrement supérieur au diamètre normal du conduit, ce qui permet, le cas échéant, d'agrandir la lumière du conduit au niveau du stent. La forme du stent dans la configuration 202 correspond à une première forme mémorisée par l'élément 210, ou première forme mémoire du stent. L'élément 210 peut être choisi tel que sa première température d'activation T2 soit supérieure à la température du corps humain ou animal, par exemple de l'ordre de 40 à 45°C, pour éviter une activation non voulue du stent pendant son insertion dans le conduit. Après sa première activation, le stent reste dans la configuration 202 même lorsque sa température redescend en dessous de la température T2, en raison des propriétés d'hystérésis du matériau à mémoire de forme.

Pour retirer le stent, on prévoit de le chauffer à une température supérieure ou égale à une deuxième température d'activation T3 de l'élément 210, supérieure à la température T2. Pour cela, un fluide chauffé à une température supérieure ou égale à la température T3 peut être injecté dans le conduit. Toutefois, dans un mode de réalisation préféré, on prévoit de chauffer le stent par effet joule en faisant circuler un courant électrique dans l'élément 210. Le courant électrique de chauffage est par exemple fourni par une pile ou une batterie couplée à un outil d'extraction du stent.

Lorsque le stent atteint la température T3, il se rétracte dans une configuration 203 dans laquelle le stent a une forme générale cylindrique de diamètre d3 inférieur au diamètre d2. Le diamètre d3 est par exemple du même ordre de grandeur que le diamètre d1 de la configuration 201, ou inférieur au diamètre d1. En tout état de cause, le diamètre d3 est inférieur au diamètre normal du conduit. La forme du stent dans la configuration 203 correspond à une deuxième forme mémorisée par l'élément 210, ou deuxième forme mémoire du stent. L'élément 210 peut être choisi tel que sa deuxième température d'activation T3 soit significativement plus élevée que la température T2, afin d'éviter une rétractation accidentelle du stent dans la configuration 203 lorsqu'on chauffe le stent pour le déployer dans la configuration 202. La température T3 est toutefois de préférence choisie suffisamment basse pour éviter des brulures du patient lors du retrait du stent. A titre d'exemple, l'élément 210 est tel que sa deuxième température d'activation T3 soit comprise dans la plage allant de 50 à 60°C. Après sa deuxième activation, le stent reste dans la configuration 203 même lorsque sa température redescend en dessous de la température T3, en raison des propriétés d'hystérésis du matériau à mémoire de forme.

Lorsque le stent est dans la configuration 203, il peut circuler librement dans le conduit ce qui permet de le retirer sans forcer et sans risque de lésion du conduit.

La figure 3 est une vue en coupe partielle illustrant plus en détail un exemple d'un mode de réalisation préféré d'un stent 300 compatible avec le fonctionnement décrit en relation avec la figure 2, c'est-à-dire capable de se déployer dans une première configuration à une première température d'activation T2 et de se rétracter dans une deuxième configuration à une deuxième température d'activation T3 supérieure à la température T2. Comme dans l'exemple de la figure 2, le stent 300 comprend un élément filiforme 310 de forme générale sensiblement hélicoïdale (non visible sur la figure 3), présentant des propriétés de mémoire de forme. Les modes de réalisation décrits ne se limitent toutefois pas à un stent de forme hélicoïdale, et d'autres formes peuvent être prévues en fonction de l'utilisation envisagée. Sur la figure 3, seule une section transversale de l'élément filiforme 310 a été représentée.

L'élément 310 comprend un fil creux ou tube 321 en un premier matériau à mémoire de forme, et, à l'intérieur du fil creux 321, un fil plein 323 en un deuxième matériau à mémoire de forme. Les fils 321 et 323 ont approximativement la même longueur, qui correspond approximativement à la longueur de l'élément 310 déroulé. Les fils 321 et 323 sont de préférence métalliques pour permettre la circulation d'un courant électrique d'activation du stent par effet joule dans l'élément 310. Les fils 321 et 323 peuvent être rendus mécaniquement et/ou électriquement solidaires l'un de l'autre à leurs extrémités et/ou sur tout ou partie de leur longueur, par exemple par frottement, soudure, etc.

A titre d'exemple, le fil creux 321 est traité pour mémoriser une forme correspondant à la configuration 202 de la figure 2 et pour s'activer à la température T2, et le fil plein 323 est traité pour mémoriser une autre forme correspondant à la configuration 203 de la figure 2 et pour s'activer à la température T3. Les traitements appliqués aux fils 321 et 323 pour obtenir les formes mémoires et les températures d'activation souhaitées ne seront pas décrits en détail ici, ces traitements étant à la portée de l'homme du métier. A titre d'exemple, on peut réaliser les fils 321 et 323 en un même matériau de départ, par exemple un même alliage nickel-titane, puis appliquer aux fils 321 et 323 des traitements thermiques différents pour modifier dans des proportions différentes les liaisons atomiques ou les compositions atomiques de l'alliage dans les deux fils pour obtenir les propriétés souhaitées.

Dans l'exemple représenté, l'élément 310 comprend en outre une gaine 325 revêtant la paroi extérieure du fil creux 321. La gaine 325 est en un matériau choisi pour empêcher ou limiter d'éventuels phénomènes d'incrustation du stent dans les parois du conduit, et faciliter l'extraction du stent. La gaine 325 est de préférence électriquement et thermiquement isolante, pour réduire les risques d'électrocution ou de brulure du patient en cas d'activation du stent par application d'un courant électrique dans le stent. La gaine 325 est par exemple en silicone. On notera que la gaine 325 peut, le cas échéant, être remplacée ou complétée par une jupe en un matériau isolant, par exemple en silicone, revêtant la paroi extérieure de la forme générale cylindrique définie par le stent.

Un avantage du mode de réalisation de la figure 3 est que le stent 300 est relativement simple à réaliser.

La figure 4 est une vue en coupe partielle illustrant un autre exemple de réalisation d'un stent 400 compatible avec le fonctionnement décrit en relation avec la figure 2. Comme dans l'exemple de la figure 2, le stent 400 comprend un élément filiforme 410 de forme générale sensiblement hélicoïdale (non visible sur la figure 4), présentant des propriétés de mémoire de forme. Sur la figure 4, seule une section transversale de l'élément filiforme 410 a été représentée.

L'élément 410 comprend un fil 421, par exemple un fil plein, présentant en section transversale deux zones 421a et 421b en des matériaux à mémoire de forme différents. Les zones 421a et 421b s'étendent par exemple sur toute la longueur du fil 421. Les modes de réalisation décrits ne se limitent toutefois pas à ce cas particulier. Le fil 421 est de préférence métallique pour permettre la circulation, dans l'élément 410, d'un courant électrique d'activation du stent par effet joule.

A titre d'exemple, la zone 421a est traitée pour mémoriser une forme correspondant à la configuration 202 de la figure 2 et s'activer à la température T2, et la zone 421b est traitée pour mémoriser une autre forme correspondant à la configuration 203 de la figure 2 et s'activer à la température T3.

A titre d'exemple, pour réaliser le fil 421, on part d'un fil en un matériau unique, par exemple un alliage nickel-titane, et on traite la totalité de ce fil pour mémoriser une première forme et s'activer à la température T2. Après ce premier traitement, le fil peut être conformé selon une deuxième forme différente de la première forme, et traité localement de façon que seule la zone 421b de la section transversale du fil mémorise la deuxième forme et acquière une température d'activation T3 supérieure à la température T2. Ce traitement localisé de la section transversale du fil est par exemple réalisé au moyen d'un faisceau laser venant chauffer un seul côté du fil sur toute la longueur du fil. D'autres procédés de traitement peuvent être envisagés pour obtenir le résultat recherché. A titre d'exemple, du nickel peut être diffusé d'un seul côté du fil. Pour cela, un élément à forte teneur en nickel peut être accolé à un seul côté du fil, le tout étant chauffé à haute température.

On notera que, bien qu'une séparation nette des zones 421a et 421b ait été représentée sur la figure 4, en pratique, en fonction du procédé de traitement utilisé, les propriétés de mémoire de forme de l'élément 410 pourront varier graduellement entre les zones 421a et 421b du fil 421.

De plus, on comprendra que le mode de réalisation de la figure 4 peut s'appliquer non seulement à un fil plein 421, comme cela vient d'être décrit, mais peut aussi s'appliquer à un fil creux.

Dans l'exemple représenté, l'élément 410 comprend en outre une gaine 425, par exemple en silicone, revêtant la paroi extérieure du fil 421.

La figure 5 est une vue en coupe selon un plan longitudinal d'un stent 500, illustrant un exemple de forme générale que peut avoir un stent du type décrit en relation avec les figures 2 à 4. Comme dans les exemples des figures 2 à 4, le stent 500 comprend un élément filiforme 510 de forme générale sensiblement hélicoïdale. Pour éviter des migrations non voulues du stent après son déploiement dans un conduit, le stent 500 a une forme en diabolo, c'est-à-dire qu'il comprend un tronçon central 501 sensiblement cylindrique, et, de part et d'autre du tronçon 501, des tronçons cylindriques 502a et 502b de diamètres supérieurs au diamètre du tronçon 501. Les modes de réalisation décrits ne se limitent toutefois pas à cet exemple particulier. D'autres formes de stent peuvent être envisagées (élargissement d'un seul côté, nombre de diamètres distincts supérieur à deux, etc.).

Un avantage des modes de réalisation décrits en relation avec les figures 2 à 5 est que les stents décrits peuvent facilement être insérés et extraits d'un conduit sans risque de lésion de ce conduit.

De plus, l'insertion et l'extraction du stent peuvent être effectuées sans nécessiter l'injection d'un fluide de chauffage ou de refroidissement dans le voisinage du stent, ce qui simplifie beaucoup les opérations de pose et de retrait du stent et évite les risques de brulure et de contamination de patient.

Les modes de réalisation décrits sont particulièrement adaptés pour une utilisation dans l'urètre lorsque le canal urétral est rétréci ou obstrué par la prostate en raison d'une hypertrophie de la prostate.

Ainsi, on prévoit d'utiliser un écarteur du type décrit en relation avec les figures 2 à 5 pour augmenter la lumière du canal urétral lorsque ce dernier est rétréci ou obstrué en raison d'une hypertrophie de la prostate.

Les modes de réalisation décrits peuvent toutefois aussi être utilisés dans d'autres canaux, conduits ou cavités du corps humain ou animal.

Dans les modes de réalisation décrits ci-dessus, des stents sont réalisés à partir d'un élément filiforme (respectivement 310 et 410 aux figures 3 et 4) comprenant, longitudinalement, deux parties (321 et 323 en figure 3 et 421a et 421b en figure 4) en des matériaux à mémoire de forme traités différemment, chaque partie mémorisant une forme du stent et s'activant (c'est-à-dire reprenant sa forme mémorisée) lorsqu'elle est chauffée à sa température d'activation.

On notera cependant que l'expression "matériau à mémoire de forme" désigne non seulement des matériaux capables de mémoriser une forme et d'y retourner lorsque leur température dépasse une température dite d'activation, mais aussi des matériaux pouvant être traités pour acquérir des propriétés dites de super-élasticité dans une certaine plage de température d'utilisation. Les matériaux à mémoire de forme traités en super-élasticités n'ont pas de température d'activation particulière, mais sont capables de subir une déformation importante de manière réversible sous l'effet d'une contrainte dans la plage de température considérée.

A titre de variante de réalisation, on peut prévoir, dans les modes de réalisations décrits ci-dessus, de réaliser l'une des deux parties à mémoire de forme du stent (par exemple la partie 321 en figure 3 et la partie 421a en figure 4) en un matériau à mémoire de forme traité pour acquérir des propriétés super-élastiques à la température du corps humain ou animal dans lequel le stent est destiné à être inséré. La super-élasticité du premier matériau peut être exploitée lors de la phase d'insertion du stent, et la forme mémorisée par le deuxième matériau peut être exploitée lors du retrait du stent.

Dans ce cas, le fonctionnement du stent est par exemple le suivant :
- Lors de son insertion dans un conduit, le stent est contraint dans une configuration correspondant à la configuration 201 de la figure 2 (déformation super-élastique), par exemple au moyen d'un outil, par exemple un cathéter, comportant des crochets permettant d'appliquer la contrainte souhaitée.
- Une fois le stent correctement positionné dans le conduit, la contrainte est relâchée, et le stent se détend, à la façon d'un ressort, pour retrouver une forme correspondant à la configuration 202 de la figure 2 (réversibilité de la déformation super-élastique).
- Pour retirer le stent, on prévoit de le chauffer à une température supérieure ou égale à la température d'activation du deuxième matériau à mémoire de forme (partie 323 du stent en figure 3 ou partie 421b du stent en figure 4). Le stent se rétracte alors dans une configuration correspondant à la configuration 203 de la figure 2, ce qui permet son retrait.

Ainsi, la pose du stent peut se faire sans chauffage, et le retrait du stent comprend une étape de chauffage.

Des modes de réalisation particuliers ont été décrits. Diverses variantes et modifications apparaîtront à l'homme de l'art.

En particulier, les modes de réalisation décrits ne se limitent pas aux structures particulières de stent décrites en relation avec les figures 2 à 5. L'homme de l'art saura adapter la solution proposée à d'autres types de stent, par exemple des stents utilisant un maillage plutôt qu'un simple élément filiforme, ou des stents comprenant un ruban hélicoïdal plutôt qu'un fil hélicoïdal.

Plus généralement, l'homme de l'art saura adapter la solution proposée à tous types d'écarteurs, dans le domaine médical ou dans d'autres domaines.

Par ailleurs, les modes de réalisation décrits ne se limitent pas au cas où les matériaux à mémoire de forme sont des alliages nickel-titane. D'autres matériaux à mémoire de forme pourront être utilisés comme par exemple certains alliages à base de cuivre et/ou d'aluminium et/ou de nickel et/ou de zinc, un alliage titane/niobium, etc.

De plus, les modes de réalisation décrits ne se limitent pas à un écarteur à mémoire de forme double. A partir de l'enseignement décrit dans la présente demande, l'homme du métier saura aussi réaliser des écarteurs dans lesquels un nombre de formes mémoires supérieur à deux est mémorisé.

## Revendications

1. Ecarteur (200 ; 300 ; 400 ; 500) comprenant un élément filiforme (210 ; 310 ; 410 ; 510) de forme générale hélicoïdale en un alliage métallique à mémoire de forme, cet élément comportant, en section transversale, des première (321 ; 421a) et deuxième (323 ; 421b) parties présentant des propriétés de mémoire de forme différentes.

2. Ecarteur (200 ; 300 ; 400 ; 500) selon la revendication 1, dans lequel la première partie (321 ; 421a) a une première température (T2) d'activation, et la deuxième partie (323 ; 421b) a une deuxième température (T3) d'activation supérieure à la première température (T2).

3. Ecarteur (200 ; 300 ; 400 ; 500) selon la revendication 2, dans lequel les première (321 ; 421a) et deuxième (323 ; 421b) parties sont agencées de façon que l'écarteur adopte une première configuration (202) lorsque la première partie (321 ; 421a) est activée, et une deuxième configuration (203) lorsque la deuxième partie (323 ; 421b) est activée.

4. Ecarteur selon la revendication 1, dans lequel la première partie présente des propriétés de super-élasticité, et la deuxième partie mémorise une forme activable thermiquement.

5. Ecarteur selon la revendication 4, dans lequel les première et deuxième parties sont agencées de façon que l'écarteur adopte une première configuration (202) lorsque la deuxième partie n'est pas activée, et une deuxième configuration (203) lorsque la deuxième partie est activée.

6. Ecarteur (200 ; 300 ; 400 ; 500) selon la revendication 3 ou 5, dans lequel au moins un tronçon de l'écarteur a une forme générale approximativement cylindrique ayant un premier diamètre (d2) dans la première configuration (202) et un deuxième diamètre (d3) inférieur au premier diamètre (d2) dans la deuxième configuration (203).

7. Ecarteur (500) selon l'une quelconque des revendications 1 à 6, dans lequel ledit élément filiforme (510) a la forme d'un diabolo.

8. Ecarteur (300) selon l'une quelconque des revendications 1 à 7, dans lequel la première partie (321) est un fil creux, et dans lequel la deuxième partie (323) est un fil de plus petit diamètre disposé dans le fil creux (321).

9. Ecarteur (400) selon l'une quelconque des revendications 1 à 7, dans lequel les première (421a) et deuxième (421b) parties sont des zones distinctes de la section transversale d'un même fil (421).

10. Ecarteur (200 ; 300 ; 400 ; 500) selon l'une quelconque des revendications 1 à 9, dans lequel ledit alliage à mémoire de forme est un alliage à base de nickel et de titane.

11. Ecarteur (200 ; 300 ; 400 ; 500) selon l'une quelconque des revendications 1 à 10, dans lequel ledit élément filiforme comprend à ses extrémités des bornes de contact permettant de faire circuler un courant électrique dans les première (321 ; 421a) et/ou deuxième (323 ; 421b) parties.

12. Ecarteur (200 ; 300 ; 400 ; 500) selon l'une quelconque des revendications 1 à 11, comprenant en outre un revêtement (325 ; 425) en silicone.

## Patentansprüche

1. Ein Abstandshalter (200; 300; 400; 500), der Folgendes aufweist:
Ein Filiformelement (210; 310; 410; 510), das eine generell schraubenförmige Form besitzt und aus einer Formgedächtnismetalllegierung hergestellt ist, wobei dieses Element im Querschnitt erste (321; 421a) und zweite (323; 421 b) Teile aufweist, die unterschiedliche Formgedächtniseigenschaften besitzen.

2. Abstandshalter (200; 300; 400; 500) nach Anspruch 1, wobei der erste Teil (321; 421 a) eine erste Aktivierungstemperatur (T2) besitzt, und der zweite Teil (323; 421 b) eine zweite Aktivierungstemperatur (T3) besitzt, die höher ist als die erste Temperatur (T2).

3. Abstandshalter (200; 300; 400; 500) nach Anspruch 2, wobei die ersten (321; 421 a) und zweiten (323; 421 b) Teile so angeordnet sind, dass der Abstandshalter eine erste Konfiguration (202) annimmt, wenn der erste Teil (321; 421 a) aktiviert ist, und eine zweite Konfiguration (203), wenn der zweite Teil (323; 421 b) aktiviert ist.

4. Abstandshalter nach Anspruch 1, wobei der erste Teil superelastische Eigenschaften besitzt und der zweite Teil eine thermisch aktivierbare Form speichert.

5. Abstandshalter nach Anspruch 4, wobei die ersten und zweiten Teile so angeordnet sind, dass der Abstandshalter eine erste Konfiguration (202) annimmt, wenn der zweite Teil nicht aktiviert ist, und eine zweite Konfiguration (203), wenn der zweite Teil aktiviert ist.

6. Abstandshalter (200; 300; 400; 500) nach Anspruch 3 oder 5, wobei wenigstens ein Abschnitt des Abstandshalters eine ungefähr im allgemeinen zylindrische Form mit einem ersten Durchmesser (d2) in der ersten Konfiguration (202) und einem zweiten Durchmesser (d3), der kleiner ist als der erste Durchmesser (d2) in der zweiten Konfiguration (203).

7. Abstandshalter (500) nach einem der Ansprüche 1 bis 6, wobei das Element (510) die Form eines Diabolos hat.

8. Abstandshalter (300) nach einem der Ansprüche 1 bis 7, wobei das erste Teil (321) ein Hohldraht ist, und wobei der zweite Teil (323) ein Draht mit einem kleineren Durchmesser ist und innerhalb des Hohldrahts (321) angeordnet ist.

9. Abstandshalter (400) nach einem der Ansprüche 1 bis 7, wobei die ersten (421 a) und zweiten (421 b) Teile unterschiedliche Bereiche des Querschnitts desselben Drahtes (421) sind.

10. Abstandshalter (200; 300; 400; 500) nach einem der Ansprüche 1 bis 9, wobei die Formgedächtnislegierung eine Legierung basierend auf Nickel und Titan ist.

11. Abstandshalter (200; 300; 400; 500) nach einem der Ansprüche 1 bis 10, wobei das Filiformelement an seinen Enden Kontaktanschlüsse aufweist, die es einem elektrischen Strom ermöglichen, in den ersten (321; 421 a) und/oder zweiten (323; 421 b) Teilen zu zirkulieren.

12. Abstandshalter (200; 300; 400; 500) nach einem der Ansprüche 1 bis 11, der ferner eine Silikonbeschichtung (325; 425) aufweist.

## Claims

1. A spacer (200; 300; 400; 500) comprising a filiform element (210; 310; 410; 510) having a generally helical shape and made of a shape-memory metal alloy, this element comprising, in cross-section, first (321; 421a) and second (323; 421b) portions having different shape-memory properties.

2. The spacer (200; 300; 400; 500) of claim 1, wherein the first portion (321; 421a) has a first activation temperature (T2), and the second portion (323; 421b) has a second activation temperature (T3) higher than the first temperature (T2).

3. The spacer (200; 300; 400; 500) of claim 2, wherein the first (321; 421a) and second (323; 421b) portions are arranged so that the spacer takes a first configuration (202) when the first portion (321; 421a) is activated, and a second configuration (203) when the second portion (323; 421b) is activated.

4. The spacer of claim 1, wherein the first portion has superelasticity properties and the second portion memorizes a thermally activatable shape.

5. The spacer of claim 4, wherein the first and second portions are arranged so that the spacer takes a first configuration (202) when the second portion is not activated, and a second configuration (203) when the second portion is activated.

6. The spacer (200; 300; 400; 500) of claim 3 or 5, wherein at least one section of the spacer has an approximately generally cylindrical shape having a first diameter (d2) in the first configuration (202) and a second diameter (d3) smaller than the first diameter (d2) in the second configuration (203).

7. The spacer (500) of any of claims 1 to 6, wherein said element (510) has the shape of a diabolo.

8. The spacer (300) of any of claims 1 to 7, wherein the first portion (321) is a hollow wire, and wherein the second portion (323) is a wire of smaller diameter arranged within the hollow wire (321).

9. The spacer (400) of any of claims 1 to 7, wherein the first (421a) and second (421b) portions are different areas of the cross-section of a same wire (421).

10. The spacer (200; 300; 400; 500) of any of claims 1 to 9, wherein said shape-memory alloy is an alloy based on nickel and titanium.

11. The spacer (200; 300; 400; 500) of any of claims 1 to 10, wherein said filiform element comprises at its ends contact terminals enabling to circulate an electric current in the first (321; 421a) and/or second (323; 421b) portions.

12. The spacer (200; 300; 400; 500) of any of claims 1 to 11, further comprising a silicone coating (325; 425).
